# EUROPEAN PATENT APPLICATION

(11) **EP 4 813 965 A1**
(43) Date of publication of application: **30.09.2026**
(21) Application number: 26166893.3
(22) Date of filing: 23.03.2026
(51) Int. Cl.: C07C 29/42

(54) **METHOD FOR PREPARING 1,4-BUTANEDIOL WITH LOW ACETAL CONTENT AND LOW 2-METHYL-1,4-BUTANEDIOL CONTENT**

(30) Priority: 25.03.2025 CN 202510357064
(71) Applicant: Shanghai DiYang Chemical Technology Co., Ltd., Shanghai 200135 (CN)
(72) Inventor: ZHANG, Qiuming, Shanghai, 200135 (CN); MA, Qiang, Shanghai, 200135 (CN); ., Agudema, Shanghai, 200135 (CN); WANG, Xudong, Shanghai, 200135 (CN); CHENG, Ru, Shanghai, 200135 (CN)
(74) Representative: Nederlandsch Octrooibureau

(57) **Abstract**

A method for producing 1,4-butanediol with low acetal content and low 2-methyl-1,4-butanediol content. The method comprises the following steps: (1) reacting acetylene with an aqueous solution of formaldehyde in the presence of a first catalyst to obtain a first reaction solution containing 2-butyne-1,4-diol; (2) subjecting the first reaction solution to rectification for formaldehyde removal, thereby obtaining a first rectified reaction solution; (3) subjecting the first rectified reaction solution to a first hydrogenation reaction with a first hydrogen stream in the presence of a second catalyst to obtain a second reaction solution containing 1,4-butanediol; (4) subjecting the second reaction solution to a second hydrogenation reaction with a second hydrogen stream in the presence of a third catalyst to obtain a third reaction solution containing 1,4-butanediol.

## Description

### Technical Field

The present disclosure belongs to the field of chemical production, and specifically relates to a method for producing 1,4-butanediol with low acetal content and low 2-methyl-1,4-butanediol content.

### Background

1,4-Butanediol (BDO) is an important organic compound. Due to its unique structure, 1,4-butanediol is widely used as an intermediate in organic synthesis, industrial solvents and polymer raw materials. Through various reactions, BDO can be converted into tetrahydrofuran (THF), γ-butyrolactone (GBL), polybutylene terephthalate (PBT), polytetramethylene ether glycol (PTMEG), N-methylpyrrolidone (NMP), etc, which is widely used in fields such as automotive manufacturing, electronic and electrical equipment, instruments and meters, household appliances and textiles, pesticides, lithium-ion batteries, plasticizers, pharmaceutical intermediates, chain extenders and adhesives.

There are multiple production routes for BDO, with the main industrialized methods being the acetylene-aldehyde method (Reppe method), maleic anhydride hydrogenation method, butadiene method and propylene oxide method. Among these BDO production methods, the Reppe method using formaldehyde and acetylene as raw materials has become the mainstream technology worldwide due to its cost advantages.

Existing the acetylene-aldehyde method units have two representative routes. The first route involves a primary reaction between acetylene and formaldehyde to produce 2-butyne-1,4-diol; this 2-butyne-1,4-diol is then subjected to two-stage hydrogenation (the two-stage hydrogenation reaction uses the same catalyst and reaction conditions) to yield BDO. Developed at an early stage, this method suffers from drawbacks such as the need for frequent catalyst replacement and significant fluctuations in product specifications corresponding to different catalyst life cycles. The second route involves a three-stage slurry bed reaction for the reaction between acetylene and formaldehyde to produce 2-butyne-1,4-diol; which is then subjected to two-stage hydrogenation to yield BDO, wherein the two hydrogenation stages utilize different catalysts and reaction conditions. This type of unit enable continuous operation without frequent shutdowns for catalyst replacement and characteristic simple operation, yet are mainly disadvantaged by a large number of reactors required. Both method routes are typical of the modified acetylene-aldehyde method BDO production methods and represent two distinct BDO quality profiles. The most prominent quality characteristic of BDO products from the first method is a high acetal content; acetal is susceptible to acidic and high-temperature conditions during downstream polymerization reactions, which impairs the product color number and restricts the application of such BDO products in the polymerization field. In contrast, BDO products from the second method have an extremely low acetal content and can be widely used in polymers such as PBT, PU/TPU. However, their high methyl BDO content limits the application of such BDO products in the electronic-grade GBL and NMP industries.

Patent Application CN109851477A holds that the formation of methyl BDO is caused by formaldehyde contained in 2-butyne-1,4-diol, and thus proposes adding a certain amount of methanol to the rectification section of 2-butyne-1,4-diol to reduce the formaldehyde content in 2-butyne-1,4-diol. This method not only increases steam consumption and rectification load but also introduces methanol, methanol requires secondary removal in the BDO rectification method, making it unsuitable for industrialization. Patent Application CN101284762A discloses a method for preparing high-purity 1,4-butanediol, which can control the methyl BDO content to below 0.07 wt% via multi-stage rectification; however, this method is flawed by high energy consumption and a complex method flow. Patent Application CN101284762A also adopts a physical separation technique: melt crystallization to obtain high-purity BDO. Although the aforementioned physical separation methods can reduce the methyl BDO content, they are associated with excessively high costs and large energy consumption, which are disproportionate to the resulting benefits.

In summary, the existing acetylene-aldehyde methods for BDO production mainly have the following drawbacks: (1) It is difficult to simultaneously reduce the contents of acetal and methyl BDO in BDO products to low levels, which impairs the overall quality of BDO products produced by the acetylene-aldehyde method; (2) Existing production methods can only remove acetal or methyl BDO individually, and the adopted conventional methods such as multi-stage rectification and melt crystallization are characterized by high energy consumption and large equipment investment. Therefore, there is an urgent need in the art to provide a method for producing 1,4-butanediol with low acetal content and low 2-methyl-1,4-butanediol content.

### Summary

An object of the present disclosure is to provide a method for producing 1,4-butanediol with low acetal content and low 2-methyl-1,4-butanediol content.

In a first aspect of the present disclosure, there is provided a method for preparing 1,4-butanediol, the method comprising the steps of:
(1) reacting acetylene with an aqueous solution of formaldehyde in the presence of a first catalyst to obtain a first reaction solution containing 2-butyne-1,4-diol;
(2) subjecting the first reaction solution to rectification for formaldehyde removal, thereby obtaining a first rectified reaction solution;
(3) subjecting the first rectified reaction solution to a first hydrogenation reaction with a first hydrogen stream in the presence of a second catalyst to obtain a second reaction solution containing 1,4-butanediol;
(4) subjecting the second reaction solution to a second hydrogenation reaction with a second hydrogen stream in the presence of a third catalyst to obtain a third reaction solution containing 1,4-butanediol;

in step (1), a molar ratio of acetylene to formaldehyde is (2-10): 1;
in step (1), a reaction pressure is 140-200 kPa;
in step (3), a molar ratio of the first hydrogen stream to 2-butyne-1,4-diol is (5-200):1;
in step (4), a molar ratio of the second hydrogen stream to 1,4-butanediol in the second reaction solution is (0.5-100):1.

In one or more embodiments, in step (1), in the aqueous solution of formaldehyde, a concentration of formaldehyde is 40-45wt%.

In one or more embodiments, in step (1), the acetylene is recycled for use in step (1).

In one or more embodiments, in step (1), the first catalyst is a supported copper catalyst.

In one or more embodiments, in step (1), a reaction temperature is 80-120 °C.

In one or more embodiments, in step (1), a reaction residence time is 0.5-200 h.

In one or more embodiments, step (1) is carried out at a pH of 3.0-5.5.

In one or more embodiments, in step (1), in the first reaction solution, a content of propargyl alcohol is ≤ 0.5 wt%.

In one or more embodiments, in step (1), in the first reaction solution, a content of formaldehyde is ≤ 1.0 wt%.

In one or more embodiments, in step (1), in the first reaction solution, a content of 2-butyne-1,4-diol is 40-45 wt%.

In one or more embodiments, a pH of step (1) is adjusted by a buffer solution, and the buffer solution comprises a strong-base weak-acid salt and an organic acid.

In one or more embodiments, in step (2), an operating pressure of a formaldehyde removal column for performing the formaldehyde removal rectification is 250-300 kPaG.

In one or more embodiments, in step (2), an operating temperature of the formaldehyde removal column for performing the formaldehyde removal rectification is 100-180 °C.

In one or more embodiments, in step (2), the formaldehyde removal column for performing the formaldehyde removal rectification is a tray column, and a number of trays in the formaldehyde removal column is 25-35.

In one or more embodiments, in step (2), a reflux ratio of the formaldehyde removal column for performing the formaldehyde removal rectification is 5-10.

In one or more embodiments, in step (2), in the first rectified reaction solution, a content of formaldehyde is ≤ 0.1 wt%.

In one or more embodiments, in step (2), in the first rectified reaction solution, a content of propargyl alcohol is ≤ 500 ppmw.

In one or more embodiments, in step (2), in the first rectified reaction solution, a content of 2-butyne-1,4-diol is 40-45 wt%.

In one or more embodiments, in step (3), a concentration of 2-butyne-1,4-diol in the first rectified reaction solution is adjusted to 35-40 wt% before the first hydrogenation reaction with the first hydrogen stream.

In one or more embodiments, step (3) is carried out in a slurry bed reactor.

In one or more embodiments, in step (3), a reaction pressure is 2.0-3.0 MPaG.

In one or more embodiments, in step (3), a reaction temperature is 40-60 °C.

In one or more embodiments, in step (3), the first hydrogen stream is recycled for use in step (3).

In one or more embodiments, step (3) is carried out at a pH of 7-11.

In one or more embodiments, in step (3), the second catalyst is a nickel catalyst.

In one or more embodiments, in step (3), a conversion rate of 2-butyne-1,4-diol is ≥ 95%.

In one or more embodiments, in step (3), a reaction time is 10-240 min.

In one or more embodiments, in step (3), in the second reaction solution, a content of 2-butene-1,4-diol is ≤ 1.0 wt%.

In one or more embodiments, in step (3), in the second reaction solution, a content of 2-methyl-1,4-butanediol is 10-2000 ppmw.

In one or more embodiments, in step (3), in the second reaction solution, a content of acetal is ≤ 600 ppmw.

In one or more embodiments, in step (4), the second reaction solution is directly used in step (4).

In one or more embodiments, in step (4), a reaction pressure is 20.0-30.0 MPaG.

In one or more embodiments, in step (4), a reaction temperature is 90-130 °C.

In one or more embodiments, in step (4), the second hydrogen stream is recycled for use in step (4).

In one or more embodiments, step (4) is carried out in a trickle bed reactor.

In one or more embodiments, in step (4), the third catalyst is a supported nickel catalyst.

In one or more embodiments, in step (4), a reaction time is 1-8 h.

In one or more embodiments, in step (4), in the third reaction solution, a content of 2-methyl-1,4-butanediol is ≤ 5000 ppmw.

In one or more embodiments, in step (4), in the third reaction solution, a content of acetal is ≤ 200 ppmw.

In one or more embodiments, the method further comprises subjecting 1,4-butanediol in the third reaction solution to a rectification post-treatment, and the rectification post-treatment comprises a dehydration method, a residue removal method, a 1,4-butanediol purification method and a 1,4-butanediol recovery method.

In one or more embodiments, the dehydration method is carried out by a dehydration system, which comprises a vacuum column and an atmospheric column.

In one or more embodiments, the residue removal method is carried out by a residue removal system, which comprises a rising film evaporator, a falling film evaporator, and a thin film evaporator.

In one or more embodiments, the 1,4-butanediol purification method is carried out by a purification system, which comprises an intermediate column and a finished product column.

In one or more embodiments, the 1,4-butanediol recovery method is carried out by a recovery system, which comprises a light component recovery column and a heavy component recovery column.

In one or more embodiments, an operating pressure of the vacuum column is 20-40 kPaA.

In one or more embodiments, an operating temperature of the vacuum column is 70-80 °C.

In one or more embodiments, the vacuum column is a tray column with 25-35 trays.

In one or more embodiments, a reflux ratio of the vacuum column is 0.5-1.0.

In one or more embodiments, an operating pressure of the atmospheric column is 35-50 kPaG.

In one or more embodiments, an operating temperature of the atmospheric column is 130-150 °C.

In one or more embodiments, an atmospheric column is a tray column, and a number of trays is 25-35.

In one or more embodiments, a reflux ratio of the atmospheric column is 1.0-1.5.

In one or more embodiments, an operating pressure of the rising film evaporator is 5-15 kPaA.

In one or more embodiments, an operating temperature of the rising film evaporator is 160-170 °C.

In one or more embodiments, an operating time of the rising film evaporator is 5-20 min.

In one or more embodiments, an operating pressure of the falling film evaporator is 5-15 kPaA.

In one or more embodiments, an operating temperature of the falling film evaporator is 160-170 °C.

In one or more embodiments, an operating time of the falling film evaporator is 5-20 min.

In one or more embodiments, an operating pressure of the thin film evaporator is 1-10 kPaA.

In one or more embodiments, an operating temperature of the thin film evaporator is 160-170 °C.

In one or more embodiments, an operating time of the thin film evaporator is 2-10 min.

In one or more embodiments, an operating pressure of the intermediate column is 3-10 kPaA.

In one or more embodiments, an operating temperature of the intermediate column is 150-170 °C.

In one or more embodiments, the intermediate column is a two-stage packed column, and heights of two packing stages are each independently 4800-5000 mm.

In one or more embodiments, a reflux ratio of the intermediate column is 25-30.

In one or more embodiments, an operating pressure of the finished product column is 1-5 kPaA.

In one or more embodiments, an operating temperature of the finished product column is 150-170 °C.

In one or more embodiments, the finished product column is a two-stage packed column, and heights of two packing stages are each independently 5400-5600 mm.

In one or more embodiments, a reflux ratio of the finished product column is 20-25.

In one or more embodiments, an operating pressure of the light component recovery column is 1-10 kPaA.

In one or more embodiments, an operating temperature of the light component recovery column is 160-170 °C.

In one or more embodiments, the light component recovery column is a two-stage packed column, and heights of two packing stages are each independently 3500-3700 mm.

In one or more embodiments, a reflux ratio of the light component recovery column is 5-15.

In one or more embodiments, an operating pressure of the heavy component recovery column is 1-5 kPaA.

In one or more embodiments, an operating temperature of the heavy component recovery column is 160-180 °C.

In one or more embodiments, the heavy component recovery column is a three-stage packed column, and heights of three packing stages are each independently 3500-4500 mm.

In one or more embodiments, a reflux ratio of the heavy component recovery column is 5-15.

In one or more embodiments, in the 1,4-butanediol prepared after the rectification post-treatment, a content of 2-(4-hydroxybutoxy)tetrahydrofuran is ≤ 150 ppmw.

In one or more embodiments, in the 1,4-butanediol prepared after the rectification post-treatment, a content of 2-methyl-1,4-butanediol is ≤ 900 ppmw.

In one or more embodiments, in the 1,4-butanediol prepared after the rectification post-treatment, a purity of 1,4-butanediol is ≥ 99.85%.

In one or more embodiments, a color number of the 1,4-butanediol prepared after the rectification post-treatment is ≤ 8.

In one or more embodiments, in the 1,4-butanediol prepared after the rectification post-treatment, a water content is ≤ 80 ppmw.

The present disclosure has the following beneficial effects:
(1) By controlling the conditions of each reaction unit (alkynylation reaction, low-pressure hydrogenation reaction, high-pressure hydrogenation reaction), the present disclosure ensures that the content of acetal entering the rectification unit (e.g., the rectification post-treatment method in Example 4) is ≤ 200 ppmw and the content of methyl BDO entering the rectification unit is ≤ 950 ppmw; the content of acetal is ≤ 150 ppmw, the content of methyl BDO is ≤ 900 ppmw, and the purity of BDO is ≥ 99.85% in the final BDO product obtained after rectification;
(2) The BDO product prepared by the method of the present disclosure can not only meet the requirements of the polymer fields with special specifications for color number (which requires low acetal content in the BDO product) such as PU/TPU, PBT and PBAT, but also be applied in the lithium-ion battery field that requires low methyl BDO content;
(3) The method of the present disclosure only needs to control the reaction conditions, without increasing additional energy consumption or modifying the rectification scheme; it is simple to operate and easy to control, suitable for large-scale industrial production, and has a good industrial application prospect.

### Detailed Description

To enable those skilled in the art to understand the characteristics and effects of the present disclosure, the following provides a general explanation and definition of the terms and expressions mentioned in the specification and claims. Unless otherwise specified, all technical and scientific terms used herein shall have the ordinary meanings understood by those skilled in the art in the context of the present disclosure; in case of a conflict, the definitions in this specification shall prevail.

Any theory or mechanism described and disclosed herein, whether correct or incorrect, shall not limit the scope of the present disclosure in any manner, that is, the content of the present disclosure may be practiced without being bound by any specific theory or mechanism.

As used herein, the terms "comprise", "include", "contain" and similar terms encompass the meanings of "consist essentially of" and "consist of'. For example, when the disclosure herein states that "A comprises B and C", the expressions "A consists essentially of B and C" and "A consists of B and C" shall be deemed to have been disclosed herein.

All characteristics defined in the form of numerical ranges or percentage ranges herein, such as values, amounts, contents and concentrations, are provided merely for brevity and convenience. Accordingly, the description of a numerical range or percentage range shall be deemed to cover and specifically disclose all possible subranges and individual numerical values (comprising integers and fractions) falling within the range.

Unless otherwise specified herein, percentages refer to weight percentages and ratios refer to weight ratios.

When describing the embodiments or examples herein, it should be understood that they are not intended to limit the present disclosure to these embodiments or examples. On the contrary, all alternatives, modifications, and equivalents of the methods and materials described in the present disclosure are intended to be encompassed within the scope defined by the claims.

For the sake of concise description, not all possible combinations of the various technical characteristics in each embodiment or example are described herein. Therefore, as long as there is no contradiction in the combination of these technical characteristics, the various technical characteristics in each embodiment or example may be combined arbitrarily, and all possible combinations shall be deemed to fall within the scope recorded in this specification.

As used herein, the term "acetal" refers to 2-(4-hydroxybutoxy)tetrahydrofuran, namely HB-THF.

As used herein, the terms "methyl butanediol" and "methyl BDO" both refer to 2-methyl-1,4-butanediol.

As used herein, the terms "butanediol" and "BDO" both refer to 1,4-butanediol.

As used herein, those skilled in the art may select the reflux ratio of each column used for rectification according to conventional methods. Since acetal and methyl BDO cannot be separated by conventional rectification, changing the reflux ratio of each rectification column has almost no effect on the separation efficiency of acetal and methyl BDO.

The inventors have found that by controlling the dosage of recycled acetylene in the alkynylation reaction, the dosage of recycled hydrogen in the low-pressure hydrogenation reaction, the dosage of recycled hydrogen in the high-pressure hydrogenation reaction, and the contents of formaldehyde and propargyl alcohol in the reaction solution at each reaction stage, butanediol with low acetal content and low methyl BDO content can be prepared through the alkynylation reaction of acetylene and formaldehyde, followed by low-pressure hydrogenation and high-pressure hydrogenation. On this basis, the present disclosure provides a method for preparing 1,4-butanediol, the method comprising the steps of:
(1) reacting acetylene with an aqueous solution of formaldehyde in the presence of a first catalyst to obtain a first reaction solution containing 2-butyne-1,4-diol;
(2) subjecting the first reaction solution to rectification for formaldehyde removal, thereby obtaining a first rectified reaction solution;
(3) subjecting the first rectified reaction solution to a first hydrogenation reaction with a first hydrogen stream in the presence of a second catalyst to obtain a second reaction solution containing 1,4-butanediol;
(4) subjecting the second reaction solution to a second hydrogenation reaction with a second hydrogen stream in the presence of a third catalyst to obtain a third reaction solution containing 1,4-butanediol.

In step (1), the concentration of formaldehyde in the aqueous solution of formaldehyde is 40-45 wt%, for example, 40 wt%, 41 wt%, 42 wt%, 43 wt%, 44 wt%, 45 wt%, and preferably 43-45 wt%. Controlling the formaldehyde concentration in the aqueous solution of formaldehyde within the range defined herein can effectively control the contents of formaldehyde and propargyl alcohol in the first reaction solution of the alkynylation reaction within the ranges defined herein, and further enable the contents of formaldehyde and propargyl alcohol in the first rectified reaction solution to fall within the ranges defined herein, which is conducive to the preparation of butanediol with low acetal content and low methyl BDO content.

The excess acetylene after the reaction with formaldehyde in step (1) is recycled for use in step (1).

In step (1), the molar ratio of acetylene to formaldehyde is (2-10): 1, for example, 2:1, 3:1, 4:1, 5:1, 6:1, 7:1, 8:1, 9:1, 10:1, and preferably (2-5):1 or (5-10):1. Controlling the molar ratio of acetylene to formaldehyde within the range defined herein can effectively control the contents of formaldehyde and propargyl alcohol in the first reaction solution of the alkynylation reaction within the ranges defined herein, and further enable the contents of formaldehyde and propargyl alcohol in the first rectified reaction solution to fall within the ranges defined herein, which is conducive to the preparation of butanediol with low acetal content and low methyl BDO content.

In step (1), the first catalyst is a supported copper catalyst. As used herein, the support of the supported copper catalyst is SiO₂ or Al₂O₃, and the active metal is copper. Based on the total mass of the first catalyst, the content of the active metal is 30-40 wt%.

Step (1) may employ a reactor conventional in the art, and is preferably carried out in a slurry bed reactor.

In step (1), the reaction temperature is 80-120 °C, for example, 80 °C, 90 °C, 100 °C, 110 °C, 120 °C, and preferably 80-90 °C or 90-120 °C. Controlling the reaction temperature of step (1) within the range defined herein can effectively control the contents of formaldehyde and propargyl alcohol in the first reaction solution of the alkynylation reaction within the ranges defined herein, and further enable the contents of formaldehyde and propargyl alcohol in the first rectified reaction solution to fall within the ranges defined herein, which is conducive to the preparation of butanediol with low acetal content and low methyl BDO content.

In step (1), the reaction pressure is 140-200 kPa, for example, 140 kPa, 150 kPa, 160 kPa, 170 kPa, 180 kPa, 190 kPa, and preferably 140-180 kPa, 140-160 kPa or 160-200 kPa. Controlling the reaction pressure of step (1) within the range defined herein can effectively control the contents of formaldehyde and propargyl alcohol in the first reaction solution of the alkynylation reaction within the ranges defined herein, and further enable the contents of formaldehyde and propargyl alcohol in the first rectified reaction solution to fall within the ranges defined herein, which is conducive to the preparation of butanediol with low acetal content and low methyl BDO content.

In step (1), the reaction residence time may be 0.5-200 h, for example, 1 h, 2 h, 4 h, 8 h, 15 h, 30 h, 50 h, 80 h, 100 h, 150 h, and preferably 0.5-4 h, 4-100 h or 4-24 h.

Step (1) is carried out at a pH of 3.0-5.5, for example, 3.2, 3.4, 3.6, 4.0, 4.3, 4.5, 4.7, 5.0, 5.1, 5.3, and preferably 4.3-4.7, 3.5-5.0 or 4.0-5.0. As used herein, the pH of the reaction system in step (1) can be adjusted by using a buffer solution. A buffer solution commonly used in the art can be selected according to the pH required for the alkynylation reaction in step (1). The buffer solution is commercially available or can be prepared in situ. The prepared buffer solution may comprise a strong-base weak-acid salt and an organic acid. Preferably, the buffer pair for adjusting the pH of the solution consists of a strong-base weak-acid salt and an organic acid. Exemplary strong-base weak-acid salt comprise, but are not limited to, sodium bicarbonate, sodium carbonate, potassium bicarbonate, potassium carbonate, sodium acetate, etc. Exemplary organic acids comprise, but are not limited to, formic acid, acetic acid, oxalic acid, citric acid, etc. Those skilled in the art can prepare a buffer solution with a specific pH according to the ionization properties of the strong-base weak-acid salt and the organic acid; for example, the molar ratio of the strong-base weak-acid salt to the organic acid may be (1-5):1, such as 1:1, 2:1, 3:1, 4:1, 5:1, and preferably (1-2):1, (2-5):1 or (2-3):1. Using the prepared buffer solution as defined in the present disclosure can control the pH of the reaction system in step (1) within the range defined herein. Controlling the pH of the reaction solution in step (1) within the range defined herein can effectively control the contents of formaldehyde and propargyl alcohol in the first reaction solution of the alkynylation reaction within the ranges defined herein, and further enable the contents of formaldehyde and propargyl alcohol in the first rectified reaction solution to fall within the ranges defined herein, which is conducive to the preparation of butanediol with low acetal content and low methyl BDO content.

In some embodiments, in step (1), the content of propargyl alcohol in the first reaction solution is ≤ 0.5 wt%, for example, 0.1 wt%, 0.2 wt%, 0.3 wt%, 0.4 wt%, 0.5 wt%, and preferably 0.1-0.5 wt% or 0.2-0.5 wt%. Controlling the content of propargyl alcohol in the first reaction solution within the range defined herein can further enable the content of propargyl alcohol in the first rectified reaction solution to fall within the range defined herein, which is conducive to the preparation of butanediol with low acetal content and low methyl BDO content.

In some embodiments, in step (1), the content of formaldehyde in the first reaction solution is ≤ 1.0 wt%, for example, 0.1 wt%, 0.2 wt%, 0.3 wt%, 0.4 wt%, 0.5 wt%, 0.6 wt%, 0.7 wt%, 0.8 wt%, 0.9 wt%, 1.0 wt%, such as 0.1-1.0 wt%, 0.3-1.0 wt% or 0.5-1.0 wt%. Controlling the content of formaldehyde in the first reaction solution within the range defined herein can further enable the content of formaldehyde in the first rectified reaction solution to fall within the range defined herein, which is conducive to the preparation of butanediol with low acetal content and low methyl BDO content.

In some embodiments, in step (1), the content of 2-butyne-1,4-diol in the first reaction solution is 40-45 wt%, for example, 40 wt%, 41 wt%, 42 wt%, 43 wt%, 44 wt%, 45 wt%, and preferably 42-44 wt% or 43-45 wt%.

In some embodiments, the first reaction solution contains impurities comprising propargyl alcohol, methanol, unreacted formaldehyde, etc.

In step (2), the operating pressure of the formaldehyde removal column for performing the formaldehyde removal rectification is 250-300 kPaG, for example, 250 kPaG, 260 kPaG, 270 kPaG, 280 kPaG, 290 kPaG, 300 kPaG, and preferably 260-280 kPaG or 270-300 kPaG. Controlling the operating pressure of step (2) within the range defined herein can effectively control the contents of formaldehyde and propargyl alcohol in the first rectified reaction solution within the ranges defined herein, which is conducive to the preparation of butanediol with low acetal content and low methyl BDO content.

In step (2), the operating temperature of the formaldehyde removal column for performing the formaldehyde removal rectification is 100-180 °C, for example, 100 °C, 110 °C, 120 °C, 130 °C, 140 °C, 150 °C, 160 °C, 170 °C, 180 °C, and preferably 100-140 °C, 130-150 °C or 140-180 °C. Controlling the operating temperature of step (2) within the range defined herein can effectively control the contents of formaldehyde and propargyl alcohol in the first rectified reaction solution within the ranges defined herein, which is conducive to the preparation of butanediol with low acetal content and low methyl BDO content.

In step (2), the formaldehyde removal column for performing the formaldehyde removal rectification is a tray column, and the number of trays of the formaldehyde removal column is 25-35, for example, 25, 26, 27, 28, 29, 30, 31, 32, 33, 34, 35, and preferably 25-30 or 30-35.

In step (2), the reflux ratio of the formaldehyde removal column for performing the formaldehyde removal rectification is 5-10, for example, 5.5, 6.0, 6.5, 7.0, 8.0, 9.0, and preferably 5-6, 5.5-10 or 5.5-8.0.

In step (2), the content of formaldehyde in the first rectified reaction solution is ≤ 0.1 wt%, for example, 0.01 wt%, 0.02 wt%, 0.05 wt%, 0.08 wt%, 0.1 wt%, and preferably 0.01-0.1 wt%. Only by controlling the content of formaldehyde in the first rectified reaction solution within the range defined herein can butanediol meeting the product specification requirements be prepared through the subsequent low-pressure hydrogenation reaction and high-pressure hydrogenation reaction.

In step (2), the content of propargyl alcohol in the first rectified reaction solution is ≤ 500 ppmw, for example, 50 ppmw, 100 ppmw, 200 ppmw, 300 ppmw, 400 ppmw, 500 ppmw, and preferably 100-500 ppmw, 150-250 ppmw or 200-500 ppmw. Only by controlling the content of propargyl alcohol in the first rectified reaction solution within the range defined herein can butanediol meeting the product specification requirements be prepared through the subsequent low-pressure hydrogenation reaction and high-pressure hydrogenation reaction.

In step (2), the content of 2-butyne-1,4-diol in the first rectified reaction solution is 40-45 wt%, for example, 40 wt%, 41 wt%, 42 wt%, 43 wt%, 44 wt%, 45 wt%, and preferably 42-44 wt% or 43-45 wt%. The rectification in step (2) mainly serves to remove formaldehyde and has little effect on the content of 2-butyne-1,4-diol in the reaction solution.

The inventors have found that under the operating conditions defined in step (2), the formaldehyde removal column can not only effectively reduce the content of formaldehyde in the first reaction solution, but also decrease the contents of other impurities (especially propargyl alcohol), while having almost no effect on the content of 2-butyne-1,4-diol in the first reaction solution.

Carrying out the alkynylation reaction of step (1) under the conditions defined herein and subjecting the reaction solution after the completion of the alkynylation reaction to the formaldehyde removal rectification of step (2) under the conditions defined herein can effectively prevent impurities such as formaldehyde and propargyl alcohol from entering the subsequent hydrogenation reactions and undergoing side reactions to generate methyl BDO. Controlling the contents of formaldehyde and propargyl alcohol in the first reaction solution within the ranges defined herein followed by the formaldehyde removal rectification defined in step (2) can effectively prevent impurities such as formaldehyde and propargyl alcohol from entering the subsequent hydrogenation reactions. If the contents of impurities (formaldehyde, propargyl alcohol, etc.) in the first reaction solution are not controlled within the ranges defined herein, the formaldehyde removal rectification in step (2) will lose its effect, resulting in excessive contents of impurities (formaldehyde, propargyl alcohol, etc.) entering the subsequent hydrogenation reactions.

Preferably, the 2-butyne-1,4-diol in the first reaction solution is subjected to formaldehyde removal rectification, and the removed formaldehyde is recovered. The recovery of formaldehyde is carried out by a formaldehyde recovery column. Those skilled in the art can select the operating conditions of the formaldehyde recovery column according to conventional chemical engineering knowledge. In some embodiments, the operating pressure of the formaldehyde recovery column is 5-20 kPaG, for example, 5-10 kPaG, 10-20 kPaG or 8-15 kPaG. In some embodiments, the operating temperature of the formaldehyde recovery column is 90-120 °C, for example, 90-105 °C, 105-120 °C or 100-110 °C. The formaldehyde recovery column is a tray column with 35-45 trays, for example, 35-40 or 40-45. The reflux ratio of the formaldehyde recovery column is 10-15, for example, 10-12 or 12-15.

In step (3), the concentration of 2-butyne-1,4-diol in the first rectified reaction solution is adjusted to 35-40 wt%, for example, 35 wt%, 36 wt%, 37 wt%, 38 wt%, 39 wt%, 40 wt%, and preferably 36-40 wt%, 35-36 wt% or 35-38 wt%, prior to conducting the first hydrogenation reaction with the first hydrogen stream. Adjusting the concentration of 2-butyne-1,4-diol in the first rectified reaction solution to 35-40 wt% for the subsequent low-pressure hydrogenation reaction enables the preparation of a second reaction solution with low acetal content and low methyl BDO content, which is conducive to the production of butanediol with low acetal content and low methyl BDO content.

Those skilled in the art may select a reactor commonly used in the art according to the reaction conditions and characteristics of step (3). In some embodiments, step (3) is carried out in a slurry bed reactor.

In some embodiments, the second catalyst is a nickel catalyst, such as a Raney nickel catalyst. As used herein, the Raney nickel catalyst refers to a Raney nickel catalyst commonly used in the art for catalytic hydrogenation. In some embodiments, the Raney nickel catalyst comprises nickel and promoters. In some embodiments, based on the total mass of the Raney nickel catalyst, the content of nickel is 50-98 wt%.

In some embodiments, in step (3), the reaction pressure is 2.0-3.0 MPaG, for example, 2.0 MPaG, 2.1 MPaG, 2.2 MPaG, 2.3 MPaG, 2.4 MPaG, 2.5 MPaG, 2.6 MPaG, 2.7 MPaG, 2.8 MPaG, 2.9 MPaG, 3.0 MPaG, and preferably 2.0-2.5 MPaG or 2.0-2.1 MPaG. Controlling the reaction pressure of step (3) within the range defined herein enables the preparation of a second reaction solution with low acetal content and low methyl BDO content, which is conducive to the production of butanediol with low acetal content and low methyl BDO content.

In some embodiments, in step (3), the reaction temperature is 40-60 °C, for example, 40 °C, 45 °C, 50 °C, 55 °C, 60 °C, and preferably 40-50 °C or 45-60 °C. Controlling the reaction temperature of step (3) within the range defined herein enables the preparation of a second reaction solution with low acetal content and low methyl BDO content, which is conducive to the production of butanediol with low acetal content and low methyl BDO content.

In some embodiments, in step (3), the reaction time is 10-240 min, for example, 20 min, 30 min, 60 min, 80 min, 120 min, 150 min, 180 min, 220 min, and preferably 30-60 min.

In some embodiments, step (3) is carried out at a pH of 7-11, for example, pH 7, 8, 9, 10, and preferably 7-9. Those skilled in the art may adjust the pH of the low-pressure hydrogenation reaction in step (3) by conventional methods, for instance, adding an alkaline reagent (such as sodium hydroxide, potassium hydroxide, etc.) to the reaction solution of step (3). The addition amount of the alkaline reagent can be calculated according to the required pH. In some embodiments, a 10-30 wt% (e.g., 20 wt%) aqueous solution of sodium hydroxide is added to the reaction solution of the low-pressure hydrogenation reaction in step (3). Controlling the pH of the reaction solution in step (3) within the range defined herein enables the preparation of a second reaction solution with low acetal content and low methyl BDO content, which is conducive to the production of butanediol with low acetal content and low methyl BDO content.

In some embodiments, in step (3), the molar ratio of the first hydrogen stream to 2-butyne-1,4-diol is (5-200):1, for example, 5:1, 10:1, 30:1, 50:1, 80:1, 100:1, 130:1, 150:1, 180:1, and preferably 10-150:1, 100-200:1 or 5-150:1. Preferably, the first hydrogen stream is recycled for use in step (3). Controlling the molar ratio of the first hydrogen stream to 2-butyne-1,4-diol in step (3) within the range defined herein enables the preparation of a second reaction solution with low acetal content and low methyl BDO content, which is conducive to the production of butanediol with low acetal content and low methyl BDO content.

In step (3), the conversion rate of 2-butyne-1,4-diol is ≥ 95%, and preferably ≥ 96%, ≥ 97%, ≥ 98% or ≥ 99%. In the low-pressure hydrogenation reaction unit, in addition to the formation of butanediol, side reactions simultaneously occur to generate various impurities such as 2-butene-1,4-diol, acetal, 4-hydroxybutyraldehyde, n-butanol, 2-(4-hydroxybutoxy)tetrahydrofuran, etc. The total content of the aforementioned organic impurities other than 2-butyne-1,4-diol (BYD) is not more than 5 wt%.

In the second reaction solution, the content of 2-butene-1,4-diol is ≤ 1.0 wt%, preferably 0.01-1.0 wt%, 0.01-0.5 wt% or 0.01-0.3 wt%. Controlling the content of 2-butene-1,4-diol in the second reaction solution within the range defined herein is conducive to the preparation of butanediol with low acetal content and low methyl BDO content.

In the second reaction solution, the content of 2-methyl-1,4-butanediol is 10-2000 ppmw, for example, 10 ppmw, 50 ppmw, 100 ppmw, 200 ppmw, 300 ppmw, 400 ppmw, 500 ppmw, 800 ppmw, 1000 ppmw, 1500 ppmw, preferably 100-1500 ppmw, 50-500 ppmw or 80-300 ppmw. Controlling the content of 2-methyl-1,4-butanediol in the second reaction solution within the range defined herein is conducive to the preparation of butanediol with low acetal content and low methyl BDO content.

In the second reaction solution, the content of acetal is ≤ 600 ppmw, for example, 50 ppmw, 100 ppmw, 200 ppmw, 300 ppmw, 400 ppmw, 500 ppmw, 600 ppmw, preferably 100-600 ppmw, 200-500 ppmw or 250-500 ppmw. Controlling the content of acetal in the second reaction solution within the range defined herein is conducive to the preparation of butanediol with low acetal content and low methyl BDO content.

Preferably, in the second reaction solution, the content of 1,4-butanediol is 30-35 wt%, for example, 30 wt%, 31 wt%, 32 wt%, 33 wt%, 34 wt%, 35 wt%, preferably 31-35 wt% or 31-33 wt%.

The second reaction solution is directly used in step (4). In the high-pressure hydrogenation unit, the second reaction solution from the low-pressure hydrogenation undergoes hydrogenation of the unreacted unsaturated organic compounds therein under the action of a third catalyst.

In some embodiments, in step (4), the reaction pressure is 20.0-30.0 MPaG, for example, 21 MPaG, 22 MPaG, 23 MPaG, 25 MPaG, 28 MPaG, and preferably 20-25 MPaG or 20-23 MPaG. Controlling the reaction pressure of step (4) within the range defined herein enables the preparation of butanediol with low acetal content and low methyl BDO content.

In some embodiments, in step (4), the reaction temperature is 90-130 °C, for example, 95 °C, 100 °C, 105 °C, 110 °C, 115 °C, 120 °C, 125 °C, 130 °C, and preferably 105-115 °C. Controlling the reaction temperature of step (4) within the range defined herein enables the preparation of butanediol with low acetal content and low methyl BDO content.

In some embodiments, in step (4), the reaction time is 1-8 h, for example, 2 h, 3 h, 5 h, 6 h, 8 h, and preferably 2-5 h.

In some embodiments, in step (4), the molar ratio of the second hydrogen stream to 1,4-butanediol in the second reaction solution is (0.5-100):1, for example, 1:1, 2:1, 5:1, 10:1, 15:1, 30:1, 50:1, 80:1, 90:1, and preferably 1-50:1, 5-30:1 or 30-50:1. Preferably, the second hydrogen stream is recycled for use in step (4). Controlling the molar ratio of the second hydrogen stream to 1,4-butanediol within the range defined herein enables the preparation of butanediol with low acetal content and low methyl BDO content.

Those skilled in the art may select a suitable reactor according to the reaction requirements. Preferably, step (4) is carried out in a trickle bed reactor. Performing step (4) in the reactor defined herein enables the preparation of butanediol with low acetal content and low methyl BDO content.

In some embodiments, the third catalyst is a supported nickel catalyst. As used herein, the support of the third catalyst is SiO₂ or Al₂O₃, and the active metal is nickel. Based on the total mass of the third catalyst, the content of the active metal is 10-20 wt%.

In the third reaction solution, the content of 2-methyl-1,4-butanediol is ≤ 5000 ppmw, for example, 50 ppmw, 100 ppmw, 200 ppmw, 300 ppmw, 400 ppmw, 500 ppmw, 600 ppmw, 800 ppmw, 1000 ppmw, 2000 ppmw, 3000 ppmw, 4000 ppmw, and preferably 50-5000 ppmw, 100-3000 ppmw, 150-2000 ppmw, 100-1000 ppmw or 150-600 ppmw. It is ensured that the content of 2-methyl-1,4-butanediol entering the rectification post-treatment unit is ≤ 950 ppmw. Controlling the content of 2-methyl-1,4-butanediol in the third reaction solution within the range defined herein enables the preparation of butanediol with low acetal content and low methyl BDO content.

In the third reaction solution, the content of acetal is ≤ 200 ppmw, for example, 10 ppmw, 20 ppmw, 50 ppmw, 80 ppmw, 100 ppmw, 120 ppmw, 150 ppmw, 180 ppmw, 200 ppmw, and preferably 10-200 ppmw, 50-190 ppmw or 80-185 ppmw. Controlling the content of acetal in the third reaction solution within the range defined herein enables the preparation of butanediol with low acetal content and low methyl BDO content.

Preferably, in the third reaction solution, the content of 1,4-butanediol is 30.1-38 wt%, for example, 31 wt%, 32 wt%, 33 wt%, 34 wt%, 35 wt%, 36 wt%, 37 wt%, 38 wt%, and preferably 31-35 wt%, 32-34 wt% or 31.1-38 wt%.

The method of the present disclosure further comprises a rectification post-treatment of 1,4-butanediol in the third reaction solution, and the rectification post-treatment comprises a dehydration method, a residue removal method, a 1,4-butanediol purification method and a 1,4-butanediol recovery method. Preferably, the method further comprises a by-product purification and recovery method. Those skilled in the art may select the operating conditions for each rectification post-treatment method according to common general knowledge in the chemical engineering field. Exemplary operating conditions for each method are described below.

The dehydration method is carried out by a dehydration system, which comprises a vacuum column and an atmospheric column; preferably, the dehydration method controls the water content in the third reaction solution to 1-3 wt%, preferably 1-1.5 wt%. The operating pressure of the vacuum column is 20-40 kPaA, for example, 23 kPaA, 25 kPaA, 27 kPaA, 30 kPaA, 35 kPaA, 37 kPaA, and preferably 23-30 kPaA or 25-30 kPaA. The operating temperature of the vacuum column is 70-80 °C, for example, 72 °C, 74 °C, 76 °C, 78 °C, and preferably 74-76 °C. The vacuum column is a tray column with 25-35 trays, for example, 26, 28, 30, 32, 34 trays, and preferably 30-35 trays. The reflux ratio of the vacuum column is 0.5-1.0, for example, 0.6, 0.7, 0.8, 0.9, and preferably 0.8-1.0. The operating pressure of the atmospheric column is 35-50 kPaG, for example, 36 kPaG, 38 kPaG, 40 kPaG, 42 kPaG, 45 kPaG, 48 kPaG, and preferably 35-40 kPaG, 40-50 kPaG or 35-45 kPaG. The operating temperature of the atmospheric column is 130-150 °C, for example, 135 °C, 140 °C, 145 °C, 150 °C, and preferably 130-140 °C, 140-150 °C or 135-145 °C. The atmospheric column is a tray column with 25-35 trays, for example, 26, 28, 30, 32, 34 trays, and preferably 30-35 trays. The reflux ratio of the atmospheric column is 1.0-1.5, for example, 1.1, 1.2, 1.3, 1.4, and preferably 1.2-1.5 or 1.0-1.2.

The residue removal method is carried out by a residue removal system, which comprises a rising film evaporator, a falling film evaporator and a thin film evaporator. The operating pressure of the rising film evaporator is 5-15 kPaA, for example, 6 kPaA, 8 kPaA, 10 kPaA, 12 kPaA, 14 kPaA, and preferably 5-10 kPaA or 10-15 kPaA. The operating temperature of the rising film evaporator is 160-170 °C, for example, 162 °C, 164 °C, 166 °C, 168 °C, and preferably 164-166 °C. The operating time of the rising film evaporator is 5-20 min, for example, 8 min, 10 min, 12 min, 15 min, 18 min, and preferably 5-10 min, 10-20 min or 5-15 min. The operating pressure of the falling film evaporator is 5-15 kPaA, for example, 6 kPaA, 8 kPaA, 10 kPaA, 12 kPaA, 14 kPaA, and preferably 5-10 kPaA or 10-15 kPaA. The operating temperature of the falling film evaporator is 160-170 °C, for example, 162 °C, 164 °C, 166 °C, 168 °C, and preferably 164-166 °C. The operating time of the falling film evaporator is 5-20 min, for example, 8 min, 10 min, 12 min, 15 min, 18 min, and preferably 5-10 min, 10-20 min or 5-15 min. The operating pressure of the thin film evaporator is 1-10 kPaA, for example, 3 kPaA, 5 kPaA, 8 kPaA, 10 kPaA, and preferably 5-10 kPaA or 1-5 kPaA. The operating temperature of the thin film evaporator is 160-170 °C, for example, 162 °C, 164 °C, 166 °C, 168 °C, and preferably 164-166 °C. The operating time of the thin film evaporator is 2-10 min, for example, 3 min, 5 min, 8 min, 10 min, and preferably 2-5 min or 5-10 min.

The 1,4-butanediol purification method is carried out by a purification system, which comprises an intermediate column and a finished product column. The operating pressure of the intermediate column is 3-10 kPaA, for example, 4 kPaA, 5 kPaA, 6 kPaA, 7 kPaA, 8 kPaA, and preferably 3-5 kPaA, 5-10 kPaA or 4-6 kPaA. The operating temperature of the intermediate column is 150-170 °C, for example, 155 °C, 160 °C, 165 °C, 170 °C, and preferably 150-160 °C, 160-170 °C or 155-165 °C. The intermediate column is a two-stage packed column, and heights of two packing stages are each independently 4800-5000 mm, for example, 4850 mm, 4900 mm, 4950 mm, 5000 mm, and preferably 4800-4900 mm, 4900-5000 mm or 4850-4950 mm. The reflux ratio of the intermediate column is 25-30, for example, 25, 26, 27, 28, and preferably 25-27. The operating pressure of the finished product column is 1-5 kPaA, for example, 1 kPaA, 2 kPaA, 3 kPaA, 4 kPaA, 5 kPaA, and preferably 1-3 kPaA, 3-5 kPaA or 2-4 kPaA. The operating temperature of the finished product column is 150-170 °C, for example, 155 °C, 160 °C, 165 °C, 170 °C, and preferably 150-160 °C, 160-170 °C or 155-165 °C. The finished product column is a two-stage packed column, and heights of two packing stages are each independently 5400-5600 mm, for example, 5450 mm, 5500 mm, 5550 mm, 5600 mm, and preferably 5500-5600 mm or 5400-5500 mm. The reflux ratio of the finished product column is 20-25, for example, 21, 22, 23, 24, 25, and preferably 22-25 or 20-22.

The 1,4-butanediol recovery method is carried out by a recovery system, which comprises a light component recovery column and a heavy component recovery column. The operating pressure of the light component recovery column is 1-10 kPaA, for example, 2 kPaA, 3 kPaA, 5 kPaA, 8 kPaA, 10 kPaA, and preferably 1-3 kPaA, 3-10 kPaA or 3-5 kPaA. The operating temperature of the light component recovery column is 160-170 °C, for example, 162 °C, 165 °C, 168 °C, and preferably 160-165 °C or 165-170 °C. The light component recovery column is a two-stage packed column, and heights of two packing stages are each independently 3500-3700 mm, for example, 3550 mm, 3600 mm, 3650 mm, 3700 mm, and preferably 3500-3600 mm or 3600-3700 mm. The reflux ratio of the light component recovery column is 5-15, for example, 6, 8, 10, 12, 14, and preferably 5-10 or 10-15. The operating pressure of the heavy component recovery column is 1-5 kPaA, for example, 1 kPaA, 2 kPaA, 3 kPaA, 4 kPaA, 5 kPaA, and preferably 1-2 kPaA or 2-5 kPaA. The operating temperature of the heavy component recovery column is 160-180 °C, for example, 163 °C, 165 °C, 167 °C, 168 °C, 169 °C, 170 °C, 175 °C, 180 °C, and preferably 165-170 °C or 167-169 °C. The heavy component recovery column is a three-stage packed column, and heights of three packing stages are each independently 3500-4500 mm, for example, 3600 mm, 3700 mm, 3800 mm, 3900 mm, 4000 mm, 4100 mm, 4200 mm, 4300 mm, 4400 mm, 4450 mm, and preferably 3600-4500 mm. The reflux ratio of the heavy component recovery column is 5-15, for example, 7, 8, 10, 12, 14, and preferably 5-10 or 10-15.

The by-product purification and recovery method is carried out by a by-product purification and recovery system, which comprises an n-butanol dehydration column and an n-butanol purification column. The operating pressure of the n-butanol dehydration column is 2-5 kPaG, and preferably 2-3 kPaG or 3-5 kPaG. The operating temperature of the n-butanol dehydration column is 95-110 °C, and preferably 95-105 °C or 105-110 °C. The n-butanol dehydration column is a tray column with a total of 20-30 trays, for example, 20-25 trays or 25-30 trays. The reflux ratio of the n-butanol dehydration column is 10-20, and preferably 10-15 or 15-20. The operating pressure of the n-butanol purification column is 3-8 kPaG, and preferably 3-5 kPaG or 5-8 kPaG. The operating temperature of the n-butanol purification column is 110-130 °C, and preferably 110-120 °C or 120-130 °C. The n-butanol purification column is a two-stage packed column, and heights of two packing stages are each independently 3500-4000 mm, for example, 3500-3760 mm or 3760-4000 mm. The reflux ratio of the n-butanol purification column is 1.0-3.0, and preferably 1.0-1.5 or 1.5-3.0.

In some embodiments, in the 1,4-butanediol prepared after rectification post-treatment, the content of 2-(4-hydroxybutoxy)tetrahydrofuran is ≤ 150 ppmw, for example, 20 ppmw, 50 ppmw, 80 ppmw, 100 ppmw, 120 ppmw, 130 ppmw, 140 ppmw, 150 ppmw, and preferably 50-150 ppmw or 80-150 ppmw.

In some embodiments, in the 1,4-butanediol prepared after rectification post-treatment, the content of 2-methyl-1,4-butanediol is ≤ 900 ppmw, for example, 20 ppmw, 50 ppmw, 80 ppmw, 100 ppmw, 120 ppmw, 150 ppmw, 300 ppmw, 400 ppmw, 500 ppmw, 600 ppmw, 700 ppmw, 800 ppmw, 900 ppmw, and preferably 50-900 ppmw, 80-800 ppmw, 100-700 ppmw or 120-650 ppmw.

In some embodiments, in the 1,4-butanediol prepared after rectification post-treatment, the purity of 1,4-butanediol is ≥ 99.85%, for example, ≥ 99.86%, ≥ 99.87%, ≥ 99.88%, ≥ 99.89%, ≥ 99.90%, ≥ 99.91%, ≥ 99.92%, ≥ 99.93%, ≥ 99.94%, ≥ 99.95%.

In some embodiments, the color number of the 1,4-butanediol prepared after rectification post-treatment is ≤ 8, for example, 2-8, 3-6, 4-5, 4.5-5.5.

In some embodiments, in the 1,4-butanediol prepared after rectification post-treatment, the water content is ≤ 80 ppmw, for example, ≤ 70 ppmw, ≤ 60 ppmw, ≤ 50 ppmw, ≤ 40 ppmw, and preferably 10-80 ppmw, 20-70 ppmw or 30-60 ppmw.

The present disclosure also provides butanediol with low acetal content and low methyl BDO content, wherein the acetal, the methyl BDO and the butanediol are as described in any embodiment herein.

The acetylene-aldehyde method for BDO production has long been criticized for inferior product quality compared with other methods such as the maleic anhydride hydrogenation method and the butadiene method. Among BDO products from different acetylene-aldehyde method routes, the quality defects associated with acetal and methyl BDO are difficult to address simultaneously. Meanwhile, with the continuous development of downstream application fields and the expansion of emerging products, the requirements for BDO product quality have become increasingly diversified, stringent and refined, posing higher demands for the quality control of BDO products produced by the acetylene-aldehyde method. Aimed at the quality defects of BDO products from the acetylene-aldehyde method, the technical solution of the present disclosure can realize low acetal content and low methyl BDO content in BDO products, improve the overall quality level of acetylene-aldehyde method BDO products, and make the products meet or even exceed the quality of those produced by methods such as the maleic anhydride hydrogenation method and the butadiene method.

The present disclosure is further illustrated below in conjunction with specific examples. It should be understood that these examples are only used to illustrate the present disclosure and not to limit the scope of the present disclosure. The experimental methods without specified conditions in the following examples are generally carried out in accordance with conventional conditions or the conditions recommended by the manufacturers. Unless otherwise stated, percentages and parts are calculated by weight.

As used herein, the content of each component in each reaction stage can be determined by chromatography, and those skilled in the art can select the test parameters of chromatography according to common general knowledge in the art.

As used herein, the abbreviations for each compound are shown in Table a below.

**Table a.**

| Compound Name | Abbreviation |
|---|---|
| 1,4-butanediol | BDO |
| 2-(4-hydroxybutoxy)tetrahydrofuran | HB-THF |
| 2-methyl-1,4-butanediol | M-BDO |
| 2-butene-1,4-diol | BED |
| n-butanol | BuOH |
| tetrahydrofuran | THF |
| γ-butyrolactone | GBL |
| 4-hydroxybutyraldehyde | HBA |
| 2-butyne-1,4-diol | BYD |
| formaldehyde | FA |
| propargyl alcohol | P₃OH |
| methanol | MeOH |

### Example 1: Alkynylation Reaction

In a slurry bed reactor, acetylene and a 45 wt% aqueous solution of formaldehyde are reacted in the presence of a copper catalyst (BASF Cu 6081P) to produce 2-butyne-1,4-diol. The molar ratio of recycled acetylene to formaldehyde in the fed formaldehyde solution is 5:1. The reaction is carried out at a temperature of 90 °C and a pressure of 160 kPa. Sodium acetate and acetic acid are fed into the reactor at a molar ratio of 2:1 to control the pH of the reaction solution at 4.5±0.2.

Recycled acetylene is discharged from the top of the slurry bed reactor after being condensed by a cooler, treated to remove organic substances in an acetylene recovery unit, and then recycled back to the slurry bed reactor for further reaction. After 4 hours of reaction, the reaction solution is monitored, and when the content of propargyl alcohol is less than 0.5 wt% and the content of formaldehyde is ≤1.0 wt%, the solution is introduced into the 2-butyne-1,4-diol rectification unit. A 43 wt% 2-butyne-1,4-diol (BYD) solution is withdrawn from the bottom of the slurry bed reactor, and is sent to the low-pressure hydrogenation reaction after subsequent 2-butyne-1,4-diol rectification.

The BYD rectification system, which consists of a column for formaldehyde removal of 2-butyne-1,4-diol and a formaldehyde recovery column, is mainly used to remove impurities such as formaldehyde and methanol, and to recover dilute formaldehyde for reuse.

The column for formaldehyde removal of 2-butyne-1,4-diol is a tray column with 30 trays, operated at a pressure of 270±10 kPaG, a temperature of 140±2 °C, and a reflux ratio of 5.5. After formaldehyde removal by rectification, the formaldehyde content in the solution is 0.05 wt%, and the propargyl alcohol content in the solution is 200±20 ppmw. Production results show that when the residual formaldehyde content in the reaction solution of the alkynylation reaction is ≤1.0 wt%, the contents of formaldehyde and propargyl alcohol in the 2-butyne-1,4-diol solution can be controlled at low levels by formaldehyde removal rectification, which is beneficial to reducing the impurity content in the final product.

Light component impurities from the top of the column for formaldehyde removal of 2-butyne-1,4-diol are sent to the formaldehyde recovery column. The formaldehyde recovery column is a tray column with 40 trays, operated at a pressure of 10±1 kPaG, a temperature of 105±2 °C, and a reflux ratio of 12.0 for formaldehyde recovery. The recovered formaldehyde can be recycled for use in the alkynylation reaction.

### Example 2: Low-Pressure Hydrogenation Reaction

Most of the BYD is converted into BDO in the low-pressure hydrogenation reaction. In Example 1, the concentration of 2-butyne-1,4-diol in the aqueous solution after formaldehyde removal by 2-butyne-1,4-diol rectification is about 43 wt%. The solution is diluted to a 36 wt% aqueous solution of 2-butyne-1,4-diol according to the requirements of the low-pressure hydrogenation reaction. The reaction is carried out in a slurry bed reactor with Raney nickel (Dalian Xi'an Technology) as the catalyst at 45±5 °C and 2.0 MPaG, and a conversion rate of 2-butyne-1,4-diol of 99% is achieved. A 20 wt% aqueous solution of sodium hydroxide is prepared and added into the reactor to control the pH of the reactor at 8±1. The content of BYD in the feed solution is determined by gas chromatography. The molar ratio of recycled hydrogen to feed BYD is 100:1. Recycled hydrogen is condensed by a cooler from the top of the low-pressure hydrogenation reactor and is then recycled back to the low-pressure hydrogenation reactor for reuse.

The low-pressure hydrogenation reaction solution is analyzed after 0.5 hour of reaction. The content of methyl BDO in the reaction solution is less than 300 ppmw, the content of acetal is ≤ 500 ppmw, and the content of 2-butene-1,4-diol is not more than 1.0 wt%. The low-pressure hydrogenation reaction is terminated, and the solution is fed to the high-pressure hydrogenation reactor. The crude BDO solution withdrawn from the bottom of the low-pressure hydrogenation reactor is further sent to the high-pressure hydrogenation reactor.

### Example 3: High-Pressure Hydrogenation Reaction

The high-pressure hydrogenation reaction is a refining hydrogenation step, which is the key step for controlling the contents of methyl BDO and acetal. The high-pressure hydrogenation reactor is a trickle bed reactor, and a supported nickel catalyst (Shanxi Jiaocheng Kate New Material Co., Ltd., grade HC08) is used. Feed hydrogen from outside the battery limits is typically provided at 3.0 MPaG and is pressurized to 30.0 MPaG by a compressor before being co-fed downflow together with the crude BDO solution into the high-pressure hydrogenation reactor from the top.

The acetal and other unsaturated compounds in the crude BDO solution are completely reacted at a reaction temperature of 110±5 °C and a reaction pressure of 20.0 MPaG. Recycled hydrogen is withdrawn below the catalyst bed of the high-pressure hydrogenation reactor, condensed by a cooler, and recycled back to the high-pressure hydrogenation reactor. The molar ratio of recycled hydrogen to BDO in the feed solution is 30:1.

After 2 hours of reaction, in the high-pressure hydrogenation reaction solution, the content of methyl BDO is determined to be less than 600 ppmw and the content of acetal to be ≤ 200 ppmw, and the reaction is terminated. The crude BDO solution withdrawn from the bottom of the high-pressure reactor is sent to the rectification post-treatment unit.

### Example 4: Rectification Post-Treatment

The crude BDO solution after high-pressure hydrogenation is subjected to five steps in the BDO rectification unit to obtain a BDO product: dehydration, residue removal, product purification, BDO recovery, and by-product purification and recovery.

The dehydration system for the dehydration step consists of a vacuum column and an atmospheric column, and is used to control the water content in the crude BDO solution to ≤ 1.5 wt%. The vacuum column is a tray column with 30 trays, operated at a pressure of 25±1.0 kPaA, a temperature of 75±1 °C, and a reflux ratio of 0.8. The atmospheric column is a tray column with 30 trays, operated at a pressure of 40±1 kPaG, a temperature of 140±1 °C, and a reflux ratio of 1.2.

The dehydrated crude BDO product is sequentially subjected to three-stage evaporation separation in a rising film evaporator, a falling film evaporator, and a thin film evaporator at 165±1 °C under high vacuum to remove residues. The rising film evaporator and the falling film evaporator are operated at a pressure of 10 kPaA for an operation time of 10 min. The thin film evaporator is operated at a pressure of 5 kPaA for an operation time of 5 min.

The BDO from which residues have been removed is purified in the product purification step. A BDO purification system composed of an intermediate column and a finished product column is adopted, and a qualified BDO product is obtained after purification. The intermediate column is a packed column packed with Sulzer BXPlus, having two packing sections with heights of 4900 mm and 4900 mm, respectively, operated at a pressure of 5±0.5 kPaA, a temperature of 160±1 °C, and a reflux ratio of 25.0. The finished product column is a packed column packed with Sulzer BXPlus, having two packing sections with heights of 5500 mm and 5500 mm, respectively, operated at a pressure of 3±0.5 kPaA, a temperature of 160±1 °C, and a reflux ratio of 22.0.

The BDO recovery step is used to recover BDO from light and heavy components discharged from the product purification step via secondary rectification to improve the yield. A light component recovery column is used to recover BDO from light components. The column is a packed column packed with Sulzer BXPlus, having two packing sections with heights of 3600 mm and 3600 mm, respectively, operated at a pressure of 3+0.5 kPaA, a temperature of 165±1 °C, and a reflux ratio of 10.0. A heavy component recovery column is a packed column packed with Sulzer BXPlus, having three packing sections with heights of 4472 mm, 3600 mm, and 3600 mm, respectively, and is used to recover BDO from heavy components, operated at a pressure of 2±0.2 kPaA, a temperature of 168±1 °C, and a reflux ratio of 10.0.

The by-product purification and recovery step is used to purify and recover n-butanol by-produced during hydrogenation, so as to obtain commercial-grade n-butanol meeting the requirements of GB/T 6027-2023. Based on the specific phase behavior of the n-butanol-water system, a system composed of an n-butanol dehydration column and an n-butanol purification column is employed. The n-butanol dehydration column is a tray column with 25 trays, operated at a pressure of 3±1 kPaG, a temperature of 105±2 °C, and a reflux ratio of 15.0. The n-butanol purification column is a two-stage packed column packed with Sulzer M252Y, having packing heights of 3760 mm and 3760 mm, respectively, operated at a pressure of 5±1 kPaG, a temperature of 120±2 °C, and a reflux ratio of 1.5.

### Example 5: Alkynylation Reaction

The only difference between Example 5 and Example 1 is that the molar ratio of recycled acetylene to fed formaldehyde is 2:1.

### Example 6: Alkynylation Reaction

The only difference between Example 6 and Example 1 is that the molar ratio of recycled acetylene to fed formaldehyde is 10:1.

### Example 7: Low-Pressure Hydrogenation Reaction

The only difference between Example 7 and Example 2 is that the molar ratio of recycled hydrogen to fed BYD is 5:1.

### Example 8: Low-Pressure Hydrogenation Reaction

The only difference between Example 8 and Example 2 is that the molar ratio of recycled hydrogen to fed BYD is 200:1.

### Example 9: Alkynylation Reaction

The only difference between Example 9 and Example 1 is that the reaction pressure is 140 kPa.

### Example 10: Alkynylation Reaction

The only difference between Example 10 and Example 1 is that the reaction pressure is 200 kPa.

### Example 11: High-Pressure Hydrogenation Reaction

The only difference between Example 11 and Example 3 is that the molar ratio of recycled hydrogen to fed BDO is 0.5:1.

### Example 12: High-Pressure Hydrogenation Reaction

The only difference between Example 12 and Example 3 is that the molar ratio of recycled hydrogen to fed BDO is 100:1.

### Comparative Example 1

The only difference between Comparative Example 1 and Example 1 is that the molar ratio of recycled acetylene to fed formaldehyde is 0.5:1.

### Comparative Example 2

The only difference between Comparative Example 2 and Example 1 is that the reaction pressure is 1000 kPa.

### Comparative Example 3

The only difference between Comparative Example 3 and Example 2 is that the molar ratio of recycled hydrogen to fed BYD is 2:1.

### Comparative Example 4

The only difference between Comparative Example 4 and Example 3 is that the molar ratio of recycled hydrogen to fed BDO is 0.3:1.

### Test Methods Used Herein

### 1. Determination of formaldehyde content.

Formaldehyde in the alkynylation reaction solution is analyzed by titration using a potentiometric titrator (Mettler Toledo T70). The specific method is as follows.

The pH of the prepared anhydrous sodium sulfite solution is adjusted to 9.5.

50 mL of the pH-adjusted anhydrous sodium sulfite solution is weighed, and a blank determination is performed using a standard sulfuric acid solution on the T70 automatic potentiometric titrator with the prescribed method.

50 mL of the sodium sulfite solution is transferred into a 100 mL titration vessel, and the reaction solution (Stage 1: 1.5±0.2 g; Stage 2: 2.5±0.2 g; Stage 3: 3±0.2 g; Rectification: 1.5±0.5 g; V402: 3±0.2 g) is transferred into the titration vessel using a Pasteur pipette. Titration is carried out to the end point with the standard sulfuric acid solution on the T70 automatic potentiometric titrator using the method "JiaQuan-D", and the value is read and recorded.

The formaldehyde content X expressed as a mass percentage is calculated by the following equation: $X = \frac{C 1 \times V 1 \times 0.03003}{M 1} \times 100$ wherein:
C₁: concentration of the standard sulfuric acid solution, mol/L;
V₁: volume of the standard sulfuric acid solution consumed in the titration, mL;
M₁: mass of the formaldehyde sample, g;
0.03003: mass per millimole of formaldehyde, g.

2. The contents of methanol, propargyl alcohol and BYD are analyzed by gas chromatography (Thermo Scientific Trace 1310) with an FID detector using the internal standard method.

3. The contents of 2-butene-1,4-diol, methanol, tetrahydrofuran, n-butanol, γ-butyrolactone, 4-hydroxybutyraldehyde, 1,4-pentanediol, butanediol, 2-methyl-1,4-butanediol, acetal and methyl pentanediol are analyzed by gas chromatography (Thermo Scientific Trace 1310) with an FID detector using the area percentage method.

4. Color number analysis.

Instrument: UV-Vis spectrophotometer, PFX-195.

Operating procedure: The colorimeter is powered on and stabilized for 30 min. A baseline program is run. A blank is prepared using distilled water or an empty cuvette. The same cuvette is rinsed 1-3 times with the sample. The quartz windows of the cuvette are wiped clean. The reading program of the colorimeter is run. The test result is recorded.

5. Test method for water content.

Water is determined by the Karl Fischer titration method (according to GB/T 6283-2008). A 0.0100 g sample is accurately weighed, added into the Karl Fischer titration medium for titration, the volume of the consumed titrant is recorded, and the water content is calculated.

6. In Tables 2-4, the term "Others" refers to other unidentified impurities other than the target product of each step and the impurities listed in the tables. The analysis is performed by gas chromatography (Thermo Scientific Trace 1310, FID detector, area percentage method).

### Test Example 1

The 2-butyne-1,4-diol solution produced in the alkynylation reaction step is tested according to the methods defined in the Test Methods Used Herein. The product specifications obtained are shown in Table 1 below.

**Table 1.**

| No. | pH | Conversion | Water | 2-butyne- 1,4-diol | Formaldehyde | Propargyl alcohol | Methanol |
|---|---|---|---|---|---|---|---|
| Unit | | wt% | wt% | wt% | wt% | wt% | wt% |
| Ex. 1 | 4.51 | 99.32 | 55.20 | 43.30 | 0.68 | 0.36 | 0.46 |
| Ex. 5 | 4.48 | 99.02 | 55.18 | 42.90 | 0.98 | 0.48 | 0.46 |
| Ex. 6 | 4.50 | 99.38 | 55.20 | 43.49 | 0.62 | 0.22 | 0.47 |
| Ex. 9 | 4.50 | 99.18 | 55.21 | 43.17 | 0.82 | 0.34 | 0.46 |
| Ex. 10 | 4.52 | 99.48 | 55.20 | 43.34 | 0.52 | 0.49 | 0.45 |
| Comp. Ex. 1 | 4.50 | 98.38 | 55.2 | 42.40 | 1.62 | 0.33 | 0.45 |
| Comp. Ex. 2 | 4.48 | 99.66 | 55.18 | 41.69 | 0.34 | 2.33 | 0.46 |

In Table 1, "Conversion" is calculated based on formaldehyde, wherein Conversion = (Feed formaldehyde - Residual formaldehyde) / Feed formaldehyde × 100%.

Methanol is introduced into the reactor together with the feed formaldehyde solution, so the methanol content remains unchanged during the reaction. A comparison of the data in Examples 1, 5 and 6 shows that adjusting the molar ratio of recycled acetylene to feed formaldehyde affects both the residual formaldehyde content and the propargyl alcohol content. As the molar ratio of recycled acetylene to feed formaldehyde increases, the propargyl alcohol content gradually decreases, thereby creating favorable conditions for reducing the methyl BDO content in the final product. A comparison of the data in Examples 1, 9, 10 and Comparative Example 2 shows that the propargyl alcohol content is positively correlated with the alkynylation reaction pressure. When the reaction pressure is further increased to 1000 kPa, the propargyl alcohol content increases sharply by approximately 50 times. Therefore, conducting the alkynylation reaction under low pressure is more conducive to controlling the propargyl alcohol content.

### Test Example 2

The crude BDO solution produced in the low-pressure hydrogenation reaction step is tested according to the methods defined in the Test Methods Used Herein. The product specifications obtained are shown in Table 2 below.

**Table 2: Analysis of Low-Pressure Hydrogenation Reaction Solution**

| No. | Unit | Ex. 2 | Ex. 7 | Ex. 8 | Comp. Ex. 3 |
|---|---|---|---|---|---|
| pH | | 8.26 | 8.28 | 8.32 | 8.32 |
| Water | wt% | 63.92 | 63.9 | 63.89 | 63.91 |
| 2-butyne-1,4-diol | wt% | 0.0032 | 0.0068 | 0.0012 | 0.0212 |
| 1,4-butanediol | wt% | 31.9018 | 31.7374 | 32.9793 | 30.7281 |
| 2-(4-hydroxybutoxy)tetrahydrofuran | ppmw | 366 | 487 | 289 | 6389 |
| 2-methyl-1,4-butanediol | ppmw | 209 | 286 | 80 | 680 |
| 2-butene-1,4-diol | wt% | 0.0238 | 0.1538 | 0.213 | 0.143 |
| n-butanol | wt% | 2.2593 | 3.2593 | 1.8657 | 1.4657 |
| tetrahydrofuran | wt% | 0.0061 | 0.0082 | 0.0022 | 0.0122 |
| γ-butyrolactone | wt% | 1.0286 | 0.0786 | 0.0171 | 1.3871 |
| 4-hydroxybutyraldehyde | wt% | 0.6953 | 0.5953 | 0.8657 | 1.2657 |
| methanol | wt% | 0.0483 | 0.051 | 0.0522 | 0.0498 |
| Others | wt% | 0.0585 | 0.1322 | 0.0766 | 0.3102 |

In Examples 2, 7 and 8, the content of 2-butyne-1,4-diol is relatively low, indicating that the low-pressure hydrogenation reaction provides a high raw material conversion rate. As can be seen from the data in the table, adjusting the molar ratio of recycled hydrogen to feed BYD affects the formation of both acetal and methyl BDO. As the molar ratio of recycled hydrogen to feed BYD increases, the acetal content and methyl BDO content gradually decrease.

### Test Example 3

The crude BDO solution produced in the high-pressure hydrogenation reaction step is tested according to the methods defined in the Test Methods Used Herein. The product specifications obtained are shown in Table 3 below.

**Table 3: Analysis of High-Pressure Hydrogenation Reaction Solution**

| No. | Unit | Ex. 3 | Ex. 11 | Ex. 12 | Comp. Ex. 4 |
|---|---|---|---|---|---|
| pH | | 8.2 | 8.24 | 8.18 | 8.14 |
| Water | wt% | 63.92 | 63.91 | 63.9 | 63.9 |
| 2-butyne-1,4-diol | wt% | Trace | Trace | Trace | Trace |
| 1,4-butanediol | wt% | 32.5521 | 32.4488 | 33.9578 | 30.5751 |
| 2-(4-hydroxybutoxy)tetrahydrofuran | ppmw | 126 | 183 | 89 | 316 |
| 2-methyl-1,4-butanediol | ppmw | 463 | 582 | 168 | 2041 |
| 2-butene-1,4-diol | wt% | 0.0008 | 0.001 | 0.0004 | 0.0026 |
| n-butanol | wt% | 2.3193 | 3.2893 | 1.9657 | 4.9657 |
| tetrahydrofuran | wt% | 0.0022 | 0.0075 | 0.0018 | 0.0088 |
| γ-butyrolactone | wt% | 1.0386 | 0.0796 | 0.0181 | 0.0813 |
| 4-hydroxybutyraldehyde | wt% | 0.0006 | 0.0023 | 0.0005 | 0.0008 |
| methanol | wt% | 0.0528 | 0.0518 | 0.0479 | 0.0504 |
| Others | wt% | 0.0547 | 0.1284 | 0.0822 | 0.1796 |

Through investigation of the reaction mechanism, the present disclosure discloses that the high-pressure hydrogenation reaction serves as a refining hydrogenation step, which mainly eliminates acetal formed as a by-product during the low-pressure hydrogenation reaction and simultaneously avoids the formation of methyl BDO. As shown by the data in Table 2 and Table 3, compared with Examples 2, 7, 8 and Examples 3, 11, 12, the acetal content in the reaction solution is significantly reduced, while the BDO content is increased. Adjusting the molar ratio of recycled hydrogen to BDO affects the formation of both acetal and methyl BDO. As the molar ratio of recycled hydrogen to BDO increases, the formation of methyl BDO is inhibited.

### Test Example 4

The product specifications are tested according to the methods defined in the Test Methods Used Herein. The test results are shown in Table 4.

Product 1 is prepared by sequentially carrying out Example 1, Example 2, Example 3 and Example 4. Product 2 is prepared by sequentially carrying out Example 1, Example 2, Example 11 and Example 4. Product 3 is prepared by sequentially carrying out Example 1, Example 2, Example 12 and Example 4.

Product 4 is prepared by sequentially carrying out Comparative Example 1, Example 2, Example 3 and Example 4. Product 5 is prepared by sequentially carrying out Comparative Example 2, Example 2, Example 3 and Example 4.

Product 6 is prepared by directly feeding the 2-butyne-1,4-diol solution obtained from the alkynylation reaction in Example 1 to the low-pressure hydrogenation reaction in Example 2 without rectification (i.e., without formaldehyde removal rectification), followed by Example 3 and Example 4.

Product 7 is prepared by sequentially carrying out Example 1, Comparative Example 3, Example 3 and Example 4.

Product 8 is prepared by sequentially carrying out Example 1, Example 2, Comparative Example 4 and Example 4.

**Table 4.**

| Item | Water | Color number | 1,4-Butanediol | HB-THF | 2-methyl-1,4-butanediol | Others |
|---|---|---|---|---|---|---|
| Unit | ppmw | APHA | wt% | ppmw | ppmw | wt% |
| Product 1 | 46 | 4.9 | 99.88 | 118 | 429 | 0.056 |
| Product 2 | 32 | 5.2 | 99.85 | 148 | 611 | 0.068 |
| Product 3 | 52 | 5 | 99.9231 | 82 | 124 | 0.051 |
| Product 4 | 48 | 4.6 | 99.58 | 596 | 2822 | 0.08 |
| Product 5 | 52 | 4.5 | 99.43 | 689 | 3604 | 0.1428 |
| Product 6 | 58 | 5 | 99.16 | 793 | 5061 | 0.26 |
| Product 7 | 50 | 5.2 | 99.41 | 1277 | 1864 | 0.28 |
| Product 8 | 55 | 5.5 | 99.63 | 312 | 1988 | 0.13 |

Analysis of the data in Table 1 (the data in Table 1 are those obtained after the alkynylation reaction without the formaldehyde removal rectification; the formaldehyde content in Comparative Example 1 exceeds the standard; the propargyl alcohol content in Comparative Example 2 exceeds the standard) and the specifications of Product 1, Product 4, Product 5 and Product 6, it can be seen that the contents of formaldehyde and propargyl alcohol in the BYD solution entering the low-pressure hydrogenation reaction have a significant influence on the contents of acetal and methyl BDO in the final BDO product. For Product 4 and Product 5, the contents of formaldehyde or propargyl alcohol in the reaction solution from the alkynylation reaction exceed the standard (see data of Comparative Example 1 and Comparative Example 2 in Table 1), which go beyond the treatment capacity of the formaldehyde removal rectification. As a result, the contents of formaldehyde or propargyl alcohol in the reaction solution fed to the subsequent hydrogenation reaction fail to meet the specification requirements of the present disclosure, which ultimately leads to the contents of acetal and methyl BDO in the prepared products far exceeding the product specifications of the present disclosure. For Product 6, since the reaction solution obtained from the alkynylation reaction in Example 1 is not subjected to the formaldehyde removal rectification, both formaldehyde and propargyl alcohol in the reaction solution fed to the low-pressure hydrogenation reaction exceed the standard, resulting in a substantial increase in acetal and methyl BDO in the final product and the worst product quality.

It can be seen from the specifications of Product 7 that when the contents of acetal and methyl BDO in the low-pressure reaction exceed the standard (see data of Comparative Example 3 in Table 2), although the acetal content in the product is reduced, it still fails to meet the low-acetal requirement, and the content of methyl BDO is more than twice the content of methyl BDO in Product 1. This indicates that although the high-pressure hydrogenation step can reduce the acetal content in the product, it has no effect on reducing the methyl BDO content. It can be seen from the specifications of Product 8 (see data of Comparative Example 4 in Table 3) that both acetal and methyl BDO in the reaction solution prepared in the high-pressure hydrogenation stage exceed the standard and do not decrease significantly after rectification in Example 4, resulting in unqualified contents of acetal and methyl BDO in the product.

In summary, it can be seen from the above comparisons that the rectification post-treatment step has little effect on reducing the contents of acetal and methyl BDO, which is also consistent with the physical property system that acetal-BDO and methyl BDO-BDO form azeotropes and have close boiling points. The specifications of the alkynylation reaction, low-pressure hydrogenation and high-pressure hydrogenation affect each other step by step. Only by controlling the specifications of each reaction can the quality of the final product be guaranteed. In the present disclosure, by controlling the reaction conditions and reaction solution specifications of the alkynylation reaction, low-pressure hydrogenation and high-pressure hydrogenation steps, the contents of acetal and methyl BDO before the rectification post-treatment step are controlled at a low level, thereby preparing a BDO product with low acetal content and low methyl BDO content.

## Claims

1. A method for preparing 1,4-butanediol, wherein the method comprises the steps of:
(1) reacting acetylene with an aqueous solution of formaldehyde in the presence of a first catalyst to obtain a first reaction solution containing 2-butyne-1,4-diol;
(2) subjecting the first reaction solution to rectification for formaldehyde removal, thereby obtaining a first rectified reaction solution;
(3) subjecting the first rectified reaction solution to a first hydrogenation reaction with a first hydrogen stream in the presence of a second catalyst to obtain a second reaction solution containing 1,4-butanediol;
(4) subjecting the second reaction solution to a second hydrogenation reaction with a second hydrogen stream in the presence of a third catalyst to obtain a third reaction solution containing 1,4-butanediol;
wherein in step (1), a molar ratio of acetylene to formaldehyde is (2-10):1;
wherein in step (1), a reaction pressure is 140-200 kPa;
wherein in step (3), a molar ratio of the first hydrogen stream to 2-butyne-1,4-diol is (5-200):1;
wherein in step (4), a molar ratio of the second hydrogen stream to 1,4-butanediol in the second reaction solution is (0.5-100): 1;
wherein in the first reaction solution, a content of propargyl alcohol is ≤ 0.5 wt%;
wherein in the first reaction solution, a content of formaldehyde is ≤ 1.0 wt%;
wherein in the third reaction solution, a content of 2-methyl-1,4-butanediol is ≤ 5000 ppmw;
wherein in the third reaction solution, a content of acetal is ≤ 200 ppmw.

2. The method according to claim 1, wherein step (1) has one or more of the following characteristics:
the acetylene is recycled for use in step (1);
the first catalyst is a supported copper catalyst;
a reaction temperature is 80-120 °C;
a reaction residence time is 0.5-200 h;
step (1) is carried out at a pH of 3.0-5.5;
preferably, in the aqueous solution of formaldehyde, a concentration of formaldehyde is 40-45 wt%;
preferably, in the first reaction solution, a content of 2-butyne-1,4-diol is 40-45 wt%.

3. The method according to claim 2, wherein a pH of step (1) is adjusted by a buffer solution, and the buffer solution comprises a strong-base weak-acid salt and an organic acid.

4. The method according to claim 1, wherein step (2) has one or more of the following characteristics:
an operating pressure of a formaldehyde removal column for performing the formaldehyde removal rectification is 250-300 kPaG;
an operating temperature of the formaldehyde removal column for performing the formaldehyde removal rectification is 100-180 °C;
the formaldehyde removal column for performing the formaldehyde removal rectification is a tray column, and a number of trays in the formaldehyde removal column is 25-35;
a reflux ratio of the formaldehyde removal column for performing the formaldehyde removal rectification is 5-10;
preferably, in the first rectified reaction solution, a content of formaldehyde is ≤ 0.1 wt%;
preferably, in the first rectified reaction solution, a content of propargyl alcohol is ≤ 500 ppmw;
preferably, in the first rectified reaction solution, a content of 2-butyne-1,4-diol is 40-45 wt%.

5. The method according to claim 1, wherein step (3) has one or more of the following characteristics:
step (3) is carried out in a slurry bed reactor;
a reaction pressure is 2.0-3.0 MPaG;
a reaction temperature is 40-60 °C;
the first hydrogen stream is recycled for use in step (3);
step (3) is carried out at a pH of 7-11;
the second catalyst is a nickel catalyst;
in step (3), a conversion rate of 2-butyne-1,4-diol is ≥ 95%;
a reaction time is 10-240 min;
preferably, a concentration of 2-butyne-1,4-diol in the first rectified reaction solution is adjusted to 35-40 wt% before the first hydrogenation reaction with the first hydrogen stream;
preferably, in the second reaction solution, a content of 2-butene-1,4-diol is ≤ 1.0 wt%;
preferably, in the second reaction solution, a content of 2-methyl-1,4-butanediol is 10-2000 ppmw;
preferably, in the second reaction solution, a content of acetal is ≤ 600 ppmw.

6. The method according to claim 1, wherein step (4) has one or more of the following characteristics:
the second reaction solution is directly used in step (4);
a reaction pressure is 20.0-30.0 MPaG;
a reaction temperature is 90-130 °C;
the second hydrogen stream is recycled for use in step (4);
step (4) is carried out in a trickle bed reactor;
the third catalyst is a supported nickel catalyst;
a reaction time is 1-8 h.

7. The method according to claim 1, wherein the method further comprises subjecting 1,4-butanediol in the third reaction solution to a rectification post-treatment, and the rectification post-treatment comprises a dehydration method, a residue removal method, a 1,4-butanediol purification method and a 1,4-butanediol recovery method.

8. The method according to claim 7, wherein the method has one or more of the following characteristics:
the dehydration method is carried out by a dehydration system, which comprises a vacuum column and an atmospheric column;
the residue removal method is carried out by a residue removal system, which comprises a rising film evaporator, a falling film evaporator, and a thin film evaporator;
the 1,4-butanediol purification method is carried out by a purification system, which comprises an intermediate column and a finished product column;
the 1,4-butanediol recovery method is carried out by a recovery system, which comprises a light component recovery column and a heavy component recovery column.

9. The method according to claim 8, wherein the method has one or more of the following characteristics:
an operating pressure of the vacuum column is 20-40 kPaA;
an operating temperature of the vacuum column is 70-80 °C;
the vacuum column is a tray column, and a number of trays is 25-35;
a reflux ratio of the vacuum column is 0.5-1.0;
an operating pressure of the atmospheric column is 35-50 kPaG;
an operating temperature of the atmospheric column is 130-150 °C;
the atmospheric column is a tray column, and a number of trays is 25-35;
a reflux ratio of the atmospheric column is 1.0-1.5;
an operating pressure of the rising film evaporator is 5-15 kPaA;
an operating temperature of the rising film evaporator is 160-170 °C;
an operating time of the rising film evaporator is 5-20 min;
an operating pressure of the falling film evaporator is 5-15 kPaA;
an operating temperature of the falling film evaporator is 160-170 °C;
an operating time of the falling film evaporator is 5-20 min;
an operating pressure of the thin film evaporator is 1-10 kPaA;
an operating temperature of the thin film evaporator is 160-170 °C;
an operating time of the thin film evaporator is 2-10 min;
an operating pressure of the intermediate column is 3-10 kPaA;
an operating temperature of the intermediate column is 150-170 °C;
the intermediate column is a two-stage packed column, and heights of two packing stages are each independently 4800-5000 mm;
a reflux ratio of the intermediate column is 25-30;
an operating pressure of the finished product column is 1-5 kPaA;
an operating temperature of the finished product column is 150-170 °C;
the finished product column is a two-stage packed column, and heights of two packing stages are each independently 5400-5600 mm;
a reflux ratio of the finished product column is 20-25;
an operating pressure of the light component recovery column is 1-10 kPaA;
an operating temperature of the light component recovery column is 160-170 °C;
the light component recovery column is a two-stage packed column, and heights of two packing stages are each independently 3500-3700 mm;
a reflux ratio of the light component recovery column is 5-15;
an operating pressure of the heavy component recovery column is 1-5 kPaA;
an operating temperature of the heavy component recovery column is 160-180 °C;
the heavy component recovery column is a three-stage packed column, and heights of three packing stages are each independently 3500-4500 mm;
a reflux ratio of the heavy component recovery column is 5-15.

10. The method according to claim 7, wherein the method has one or more of the following characteristics:
in the 1,4-butanediol prepared after the rectification post-treatment, a content of 2-(4-hydroxybutoxy)tetrahydrofuran is ≤ 150 ppmw;
in the 1,4-butanediol prepared after the rectification post-treatment, a content of 2-methyl-1,4-butanediol is ≤ 900 ppmw;
in the 1,4-butanediol prepared after the rectification post-treatment, a purity of 1,4-butanediol is ≥ 99.85%;
a color number of the 1,4-butanediol prepared after the rectification post-treatment is ≤ 8;
in the 1,4-butanediol prepared after the rectification post-treatment, a water content is ≤ 80 ppmw.

11. The method according to claim 2, wherein a support of the supported copper catalyst is SiO₂ or Al₂O₃, and an active metal is copper; preferably, based on a total mass of the supported copper catalyst, a content of the active metal is 30-40 wt%.

12. The method according to claim 5, wherein the nickel catalyst is a Raney nickel catalyst.

13. The method according to claim 6, wherein a support of the supported nickel catalyst is SiO₂ or Al₂O₃, and an active metal is nickel; preferably, based on a total mass of the supported nickel catalyst, a content of the active metal is 10-20 wt%.

14. The method according to claim 3, wherein the strong-base weak-acid salt is selected from one or more of sodium bicarbonate, sodium carbonate, potassium bicarbonate, potassium carbonate and sodium acetate; and/or the organic acid is selected from one or more of formic acid, acetic acid, oxalic acid and citric acid.

15. Use of controlling a molar ratio of acetylene to formaldehyde in step (1) to (2-10):1, a molar ratio of a first hydrogen stream to 2-butyne-1,4-diol in step (3) to (5-200):1, a molar ratio of a second hydrogen stream to 1,4-butanediol in a second reaction solution in step (4) to (0.5-100):1, a content of propargyl alcohol in a first reaction solution to ≤ 0.5 wt%, and a content of formaldehyde in the first reaction solution to ≤ 1.0 wt%, in a method for preparing 1,4-butanediol, for reducing a content of acetal and/or 2-methyl-1,4-butanediol in an obtained 1,4-butanediol product;
wherein the method for preparing 1,4-butanediol comprises the steps of:
(1) reacting acetylene with an aqueous solution of formaldehyde in the presence of a first catalyst to obtain a first reaction solution containing 2-butyne-1,4-diol;
(2) subjecting the first reaction solution to rectification for formaldehyde removal, thereby obtaining a first rectified reaction solution;
(3) subjecting the first rectified reaction solution to a first hydrogenation reaction with a first hydrogen stream in the presence of a second catalyst to obtain a second reaction solution containing 1,4-butanediol;
(4) subjecting the second reaction solution to a second hydrogenation reaction with a second hydrogen stream in the presence of a third catalyst to obtain a third reaction solution containing 1,4-butanediol.
